# EUROPÄISCHE PATENTSCHRIFT

(11) **EP 2 536 440 B1**
(45) Veröffentlichungstag und Bekanntmachung des Hinweises auf die Patenterteilung: **11.05.2016**
(21) Anmeldenummer: 11702003.2
(22) Anmeldetag: 27.01.2011
(51) Int. Cl.: A61L 2/18, A61L 2/24, A61C 19/00, A61L 2/07

(54) **GERÄT ZUM DESINFIZIEREN, STERILISIEREN UND/ODER PFLEGEN VON ÄRZTLICHEN, INSBESONDERE ZAHNÄRZTLICHEN INSTRUMENTEN**
DEVICE FOR DISINFECTING, STERILIZING AND/OR MAINTAINING MEDICAL, ESPECIALLY DENTAL, INSTRUMENTS
APPAREIL DE DÉSINFECTION, DE STÉRILISATION ET/OU D'ENTRETIEN D'INSTRUMENTS MÉDICAUX, NOTAMMENT DENTAIRES

(30) Priorität: 17.02.2010 DE 102010002027
(43) Veröffentlichungstag der Anmeldung: 26.12.2012
(73) Patentinhaber: Kaltenbach & Voigt GmbH, 88400 Biberach (DE)
(72) Erfinder: HECKENBERGER, Hans, 88433 Aßmannshardt (DE); WIEK, Hans-Dieter, 88454 Hochdorf 2 (DE)
(74) Vertreter: Thun, Clemens
(86) Internationale Anmeldenummer: PCT/EP2011/051121
(87) Internationale Veröffentlichungsnummer: WO 2011/101214

(56) Entgegenhaltungen:
- EP-A1- 0 638 297
- EP-A1- 1 749 502
- WO-A2-2010/106161
- DE-U1- 20 017 039

## Beschreibung

Die vorliegende Erfindung betrifft ein Gerät, welches zum Desinfizieren, Sterilisieren und/oder Pflegen von ärztlichen Instrumenten vorgesehen ist. Insbesondere sollen mit dem Gerät zahnärztliche Instrumente aufbereitet werden.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führen die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Aus den zuvor genannten Gründen werden in letzter Zeit verstärkt Geräte bzw. Systeme eingesetzt, welche eine vollständige Aufbereitung eines zahnärztlichen Instruments ermöglichen. Diese Geräte führen aufeinanderfolgend verschiedene Maßnahmen durch, um die Instrumente aufzubereiten, wobei sie auf unterschiedlichste Medien zurückgreifen. Beispielsweise werden Reinigungsmittel, Desinfektionsmittel und Pflege- und Druckluft eingesetzt, um eine vollständige Wiederaufbereitung der Instrumente zu erzielen. Dies erfordert zwangsläufig, dass ein System zum Verteilen und zur Verfügung stellen unterschiedlichster Medien vorhanden ist.

Bislang bekannte Lösungen basieren darauf, dass die einzelnen Medien durch entsprechende Magnetventilblöcke auf die Instrumente verteilt werden. Eine derartige Lösung ist mit einem verhältnismäßig hohen Aufwand und demzufolge auch mit entsprechenden Kosten verbunden.

Aus der EP 1 749 502 A1 ist ein Pflege- oder Reinigungsgerät für medizinische Instrumente bekannt, bei dem Wasser und ein Reinigungsmittel über Leitungen den Instrumenten zugeführt werden. In den Leitungen befinden sich Ventile.

Aus der EP 0 638 297 A1 ist eine Vorrichtung zur hygienischen Aufbereitung von zahnmedizinischen Instrumenten bekannt, bei der den Instrumenten über Magnetventile Luft, Wasser und Öl zugeführt werden. Zur Steuerung der Magnetventile dient ein Mikroprozessor.

Der vorliegenden Erfindung liegt deshalb die Aufgabe zugrunde, eine kostengünstige Alternative zum Bereitstellen verschiedener Medien zur Verfügung zu stellen.

Die Aufgabe wird durch ein Wiederaufbereitungsgerät zum Desinfizieren, Sterilisieren und/oder Pflegen von ärztlichen, insbesondere zahnärztlichen Instrumenten, welches die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf der Nutzung einer neuartigen Verteilereinrichtung, welche speziell ausgestaltete und kostengünstig herzustellende Ventile aufweist. Hierzu ist vorgesehen, dass jedes Ventil ein Dichtelement aufweist, dass durch ein wahlweise positionierbares Betätigungselement der Verteilereinrichtung in eine das Ventil öffnende Stellung bringbar ist.

Dementsprechend wird gemäß der vorliegenden Erfindung ein Wiederaufbereitungsgerät zum Desinfizieren, Sterilisieren und/oder Pflegen von ärztlichen, insbesondere zahnärztlichen Instrumenten vorgeschlagen, welches eine Verteilereinrichtung zum Zuführen von Reinigungs- oder Pflegemedien zu den Instrumenten aufweist, wobei die Verteilereinrichtung für jedes Medium einen zentralen Zugang aufweist, der jeweils über ein Ventil mit einer Halterung für ein Instrument verbunden ist, und wobei jedes Ventil ein Dichtelement aufweist, dass durch ein wahlweise positionierbares Betätigungselement der Verteilereinrichtung in eine das Ventil öffnende Stellung bringbar ist.

Das Betätigungselement ist an einem Schieber angeordnet, der beispielsweise linear bewegbar oder drehbar sein kann. Das Dichtelement eines Ventils kann aus einem ferritischem Material bestehen. Das Betätigungselement ist durch einen Magneten gebildet, insbesondere durch einen Elektromagneten, der wahlweise an der Oberseite einer Verteilerplatte positioniert werden kann, wobei die Positionierung des Magneten über den Schieber erfolgt.

Das Dichtelement wiederum kann durch einen Stößel oder durch eine Platte gebildet sein.

Letztendlich wird durch die erfindungsgemäße Lösung eine Anordnung geschaffen, mit der in einfacher und komfortabler Weise die verschiedenen Medien zu den aufzubereitenden Instrumenten geleitet werden können. Diese Anordnung ist kostengünstig realisierbar, weist allerdings trotz allem eine hohe Betriebssicherheit auf.

Nachfolgend soll die Erfindung anhand der beiliegenden Zeichnung näher erläutert werden. Es zeigen:
- Figur 1: in Schnittdarstellung eine Prozess- bzw. Spülkammer eines Gerätes zum Desinfizieren, Sterilisieren und/oder Pflegen von zahnärztlichen Instrumenten;
- Figur 2: ein einzelnes Ventil einer erfindungsgemäßen Anordnung zum Zuführen von Medien und
- Figur 3: schematisch die Ausgestaltung einer Anordnung zum Zuführen verschiedener Medien.

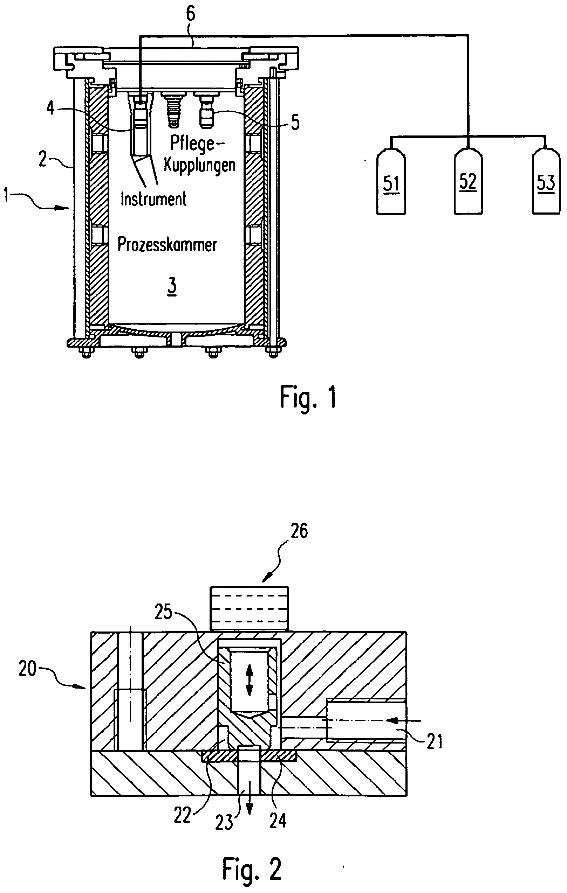

Figur 1 zeigt zunächst schematisch die Ausgestaltung eines Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist. Zentrales Element des erfindungsgemäßen Pflegegeräts 1 ist ein Druckbehälter 2, der eine Prozess- bzw. Spülkammer 3 umschließt. In dieser Spülkammer 3 sind während des Prozessablaufs die zu reinigenden bzw. pflegenden Instrumente 4 angeordnet. Die Anordnung der Instrumente 4 erfolgt hierbei mit Hilfe eines Instrumententrägers, auf dem mehrere Steckplätze bzw. Kupplungen 5 angeordnet sind. Vorzugsweise sind unterschiedliche Kupplungen 5 vorgesehen, sodass Instrumente 4 mit Kupplungssystemen verschiedener Hersteller aufbereitet werden können. Als Instrumententräger dient im vorliegenden Fall der Deckel 6 der Prozesskammer 3. Dieser Deckel 6 stellt die fluidische Ankopplung der zu reinigenden Instrumente 4 an ein Versorgungssystem sicher. Er wird durch eine Verriegelungsvorrichtung auf den Bund des Druckbehälters 2 geklemmt und gegenüber diesem abgedichtet. Über in den Deckel 6 integrierte Verbindungsrohre können dann die einzelnen Instrumente 4 und deren Kanäle einzeln oder gemeinsam mit einem Reinigungs- und/oder Pflegemittel beaufschlagt werden.

Nachfolgend soll zunächst allgemein der Prozessablauf bei der Reinigung und/oder Pflege der Instrumente 4 beschrieben werden. Hierbei wird vor dem Start der Aufbereitung die Druckdichtigkeit der Prozesskammer 3 überprüft. Dabei wird sichergestellt, dass der Deckel 6 richtig eingesetzt und mit dem Druckbehälter 2 verriegelt ist. Auch eine korrekte Verbindung der Fluidleitungen zwischen dem Deckel 6 und in dem Bund des Druckbehälters 2 verlaufenden Leitungen wird überprüft.

Zur Wasserversorgung des Geräts 1 wird Leitungswasser vorzugsweise mittels einer Osmose-Anlage mit oder ohne nachgeschalteten Mischbett-Ionenaustauscher filtriert, wobei die gelösten Salze entfernt werden. Das Wasser bei einer Qualität von <15 µS/cm wird in einen geräteseitigen Vorratsbehälter geleitet, wobei der Füllstand über einen Niveauschalter, der als Schwimmerschalter ausgestaltet ist, und die Güte über einen Leitwertsensor kontrolliert wird. Der Einlass in den Vorratsbehälter ist aus hygienischen Gründen mit einer sogenannten freien Fallstrecke ausgestaltet.

Beim Aufbereiten der Instrumente mit Hilfe des erfindungsgemäßen Geräts werden dann nacheinander die folgenden Schritte ausgeführt:
a) Reinigen
   Zunächst wird Wasser aus dem zuvor beschriebenen Vorratsbehälter in die Prozesskammer 3 geleitet, wobei dies über eine Pumpe oder durch Ansaugen über Vakuum erfolgen kann. In der Prozesskammer 3 wird das Wasser mit Hilfe von Heizelementen auf etwa 45°C aufgeheizt. Dabei wird darauf geachtet, dass die Temperatur nicht oberhalb von 45°C liegt, um ein Koagulieren von Eiweiß zu verhindern. Das Wasser wird ferner mit Hilfe einer Pumpe umgewälzt und über Sprühdüsen, welche an der Mantelfläche des Druckbehälters 2 oder in einem Zentraldom angebracht sind, auf die Außenflächen der Instrumente 4 gerichtet, um diese zu reinigen. Dabei kann das Reinigungswasser durch die Instrumente 4 und/oder die Spraykanäle der Instrumente 4 und/oder zur Außenreinigung durch die Sprühdüsen der Prozesskammer 3 geleitet werden.
   Das Aufheizen des Waschmediums kann während der Umwälzung erfolgen, sodass die zu reinigenden Flächen zunächst mit kaltem Waschmedium gereinigt werden. Das Reinigungsmittel kann hierbei in Form von Pulver oder in Tablettenform in die Prozesskammer 3 zugegeben werden oder aus einem entsprechenden Vorratsbehälter zudosiert werden. Das Waschmedium kann dabei aus Tensiden bzw. Phosphaten bestehen und einen ph-Wert oberhalb von 10 aufweisen. Zur Beendigung des Waschvorgangs wird das Wasser aus dem Druckbehälter 2 abgelassen.
b) Klarspülen - Neutralisation
   In einem darauffolgenden Schritt wird dann das Wasser aus dem Vorratsbehälter in die Prozesskammer 3 geleitet und nun auf ca. 45°C bis 60°C aufgeheizt. Während des Umwälzenz des Wassers wird aus einem weiteren Vorratsbehälter Klarspüler bzw. Neutralisator zudosiert. Alternativ kann aufgrund der höheren Temperatur im Vergleich zu dem Schritt a) nunmehr auch eine zweite Komponente einer Reinigungstablette aufgelöst werden. Die Flüssigkeit wird wiederum parallel oder zeitversetzt bzw. im Intervallbetrieb durch die Instrumente 4 und die Spraykanäle geleitet bzw. über die Sprühdüsen auf die Außenflächen der Instrumente 4 gerichtet. Als Klarspüler bzw. Neutralisator kommt insbesondere Phosphorsäureester mit einem ph-Wert von 3 bis 5 zur Anwendung.
   Die Flüssigkeit kann wiederum aus dem Druckbehälter in die Kanalisation abgelassen werden oder verbleibt im Behälter, um beim späteren Pflegevorgang aus den Instrumenten 4 austretendes überschüssiges Pflegemittel aufzunehmen bzw. um die ölige Instrumentenaußenfläche mit warmer Flüssigkeit kurz abzuspülen. In diesem Fall wird die Flüssigkeit erst nach dem Pflegevorgang abgelassen, wobei es hilfreich sein kann, die Instrumente 4 mit Druckluft zu beaufschlagen, um ein Eindringen von Sprühwasser in das Innere der Instrumente 4 zu verhindern.
c) Pflegen
   In einem dritten Schritt wird aus einem Pflegemittelvorratsbehälter Pflegemittel in das Instrumenteninnere geleitet, sodass die Getriebe und Lagerstellen geschmiert werden. Das Pflegemittel kann dabei in flüssiger Form als Öl oder aus einer Druckdose in einen Druckluftstrahl injektiert werden. Es ist auch möglich, das Öl über das in der Druckdose enthaltene Treibmittel aufzuschäumen und das Instrumenteninnere mit diesem Öl-Luft-Schaum zu füllen. Die Luftbläschen kollabieren in diesem Fall verhältnismäßig rasch, sodass das Öl im gesamten Instrumenteninneren einen gleichförmige dünnen Ölfilm bildet. Als Schmierstoffe kommen biologisch abbaubare Fettsäure-Esteröl/Weißöl-Gemische zur Anwendung.
d) Abspülen
   Nachdem zuvor beschriebenen Pflegevorgang können die Instrumente an der Außenfläche mit der noch im Behälter befindlichen Klarspülflüssigkeit abgespült werden. Alternativ hierzu wird über eine Pumpe frisches Wasser aus dem Vorratsbehälter der Prozesskammer 3 zugeführt und über die Sprühdüsen auf die Instrumentenaußenflächen gerichtet.
e) Sterilisation - Vorvakuum
   Zum Sterilisieren der Instrumente wird der Prozesskammer 3 frisches Wasser aus dem Vorratsbehälter zugeführt. In der Prozesskammer 3 ist zur Entlüftung eine Vakuumeinrichtung angeschlossen, wobei der Druck innerhalb der Prozesskammer 3 überwacht bzw. registriert wird.
   Mit Hilfe der Vakuumeinrichtung wird die Luft aus der Prozesskammer 3 abgesaugt. Das Vakuum wird durch Aufheizen des Wassers über Heizelemente bis auf Atmosphärendruck abgebaut. Die Prozesskammer 3 wird dann mit Wasserdampf befüllt, wobei sich dieser Vorgang je nach Sterilisationsprogramm mehrmals wiederholen kann.
   Das verdampfte Wasservolumen kann bei jedem Vakuumzyklus nachgefüllt werden, wobei alternativ hierzu auch die gesamte für die Dampferzeugung erforderliche Wassermenge gleich zu Beginn des Sterilisationszyklus in die Prozesskammer 3 eingebracht werden kann.
   Alternativ zur Dampferzeugung über in der Prozesskammer 3 befindliche Heizelemente kann Wasserdampf zum Druckausgleich bei der Entlüftung bzw. zur Sterilisation auch aus einem außerhalb der Prozesskammer 3 befindlichen Dampfdruckkessel zugeführt werden.
f) Trocknung und Kühlung
   Nach Abschluss der Sterilisation werden die Instrumente 4 getrocknet, in dem der in der Prozesskammer 3 befindliche Wasserdampf zur Kondensation gebracht wird. Dies wird dadurch erzielt, dass die Behälterwand oder in dem Behälter befindliche Elemente gekühlt werden, beispielsweise indem aus dem Vorratshälter entnommenes Wasser durch sie geleitet wird. Dabei kann das Wasser kontinuierlich oder intervallförmig zugeführt werden. Nach Beendigung des Kühlvorgangs wird das Wasser abgeleitet. Da nunmehr innerhalb der Kamer 3 eine Temperatur unterhalb von 50°C vorliegt, kann der Deckel 6 geöffnet werden. Der Aufbereitungszyklus für die Instrumente 4 ist hierdurch abgeschlossen.

Aus der obigen Schilderung ergibt sich, dass mit dem Gerät 1 eine vollautomatische Aufbereitung zahnärztlicher Instrumente ermöglicht wird. Eingriffe durch Bedienpersonal sind nicht erforderlich, sodass ein sehr komfortables System vorliegt. Dabei kann selbstverständlich auch von dem geschilderten Auflauf zum Aufbereiten der Instrumente abgewichen werden. Gleichzeitig ist erkennbar, dass unterschiedlichste Mittel und Medien zum Aufbereiten der Geräte genutzt werden.

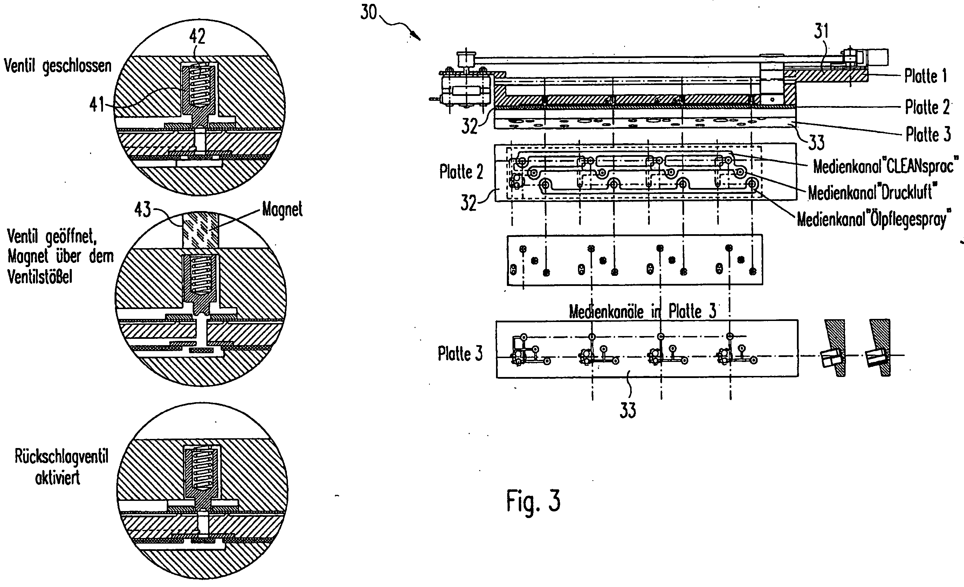

Wie in Figur 1 schematisch dargestellt ist, ist dementsprechend das Gerät 1 mit verschiedenen Vorratsbehältern für unterschiedliche Medien verbunden, wobei die in den Vorratsbehältern befindlichen Medien wahlweise entnommen und den Instrumenten zugeführt werden. Hierfür ist eine Anordnung zum Verteilen der verschiedenen Medien verantwortlich, welche bislang in erster Linie auf der Nutzung mehrerer Magnetventilblöcke beruhte, über welche die Medien auf die Instrumente verteilt wurden. Mit der vorliegenden Erfindung wird nunmehr eine neuartige Anordnung zum Verteilen der Medien vorgeschlagen, welche einfacher und kostengünstiger realisiert werden kann. Diese soll nachfolgend anhand der Figuren 2 und 3 erläutert werden.

Die erfindungsgemäße Anordnung beruht auf speziell ausgestalteten Ventilen, wobei ein Ventil hiervon in Figur 2 dargestellt ist. Es weist einen Medieneingang 21 auf, der über eine Kammer 22 mit dem Ausgang 23 des Ventils 20 verbunden ist. Am Ausgang befindet sich eine Dichtung 24, welche über einen Stößel 25 abgedichtet wird. Der Stößel 25 kann wahlweise in eine das Ventil 20 öffnende oder schließende Position bewegt werden.

Das Betätigen des Stößels 25 erfolgt über ein an der Oberseite des Ventils angeordnetes Betätigungselement 26, das im vorliegenden Fall durch einen Magneten gebildet ist. Dieser Magnet 26 kann - wie nachfolgend näher erläutert wird - wahlweise an der Oberseite einer so genannten Verteilerplatte positioniert werden. Befindet sich der Magnet 26, der beispielsweise durch einen Elektromagneten gebildet sein kann, oberhalb des Stößels 25, so wird der - vorzugsweise aus einem ferritischen Material bestehende Stößel 25 - durch den magnetischen Fluss angezogen. Er hebt von der Dichtung 24 ab und gibt dadurch den Weg für das zu schaltende Medium frei.

Der obigen Schilderung ist entnehmbar, dass ein sehr einfaches Ansteuern des Ventils 20 ermöglicht ist. Dieses Grundprinzip wird nunmehr zur Realisierung einer größeren Anordnung zum Verteilen der Medien genutzt, welche in Figur 3 dargestellt ist. Diese Anordnung soll im dargestellten Ausführungsbeispiel dazu dienen, drei verschiedene Medien wahlweise den Anschlussstellen für vier zahnärztliche Instrumente zuzuführen.

Die Anordnung 30 besteht im Wesentlichen aus drei übereinander angeordneten Platten, wobei in der oberen Platte 31, der so genannten Verteilerplatte, die Stößel der verschiedenen Ventile angeordnet sind. In der darunter liegenden zweiten Platte 32 sind Medienkanäle ausgebildet, die jeweils von einem zentralen Zugang, der mit dem entsprechenden Vorratsbehälter verbunden ist, zu vier Entnahmestellen verzweigen. Unterhalb der Medienkanäle sind dann die zuvor angesprochenen Dichtungen für die einzelnen Ventile angeordnet, wobei im vorliegenden Fall die Dichtungen mit integrierten Rückschlagventilen ausgebildet sind. Unterhalb der Dichtungen ist die dritte Platte angeordnet, in der wiederum einzelne Kanäle ausgebildet sind, die von den Ventilöffnungen jeweils zu dem zugehörigen Kupplungsadapter für das wiederaufzubereitende Instrument führen.

Durch die Anordnung werden also insgesamt zwölf Ventile geschaffen, über die die verschiedenen Medien wahlweise den Instrumenten zugeführt werden können. Die Ventile sind dabei im Ausgangszustand immer geschlossen, was dadurch erreicht wird, dass - wie in der linken Seite von Figur 3 angedeutet - der Stößel 41 jeweils durch ein federndes Element, beispielsweise eine Spiralfeder 42 in die Schließposition gedrückt wird. Durch die Anordnung eines Magneten 43 an der Oberseite des Stößels 41 kann allerdings das Ventil wahlweise geöffnet werden. Die Positionierung des Magneten 43 erfolgt hierbei über einen Schieber, der entweder linear bewegt werden kann oder drehbar ist. Dieser Schieber ermöglicht es, den Magneten 43 zum Betätigen des Ventils wahlweise an der entsprechenden Position anzuordnen und dadurch den Weg für das zu schaltende Medium freizugeben. Wird hingegen der Magnet 43 wieder entfernt, so wird der Stößel 41 durch die Feder 42 wieder auf die Dichtung gepresst.

Die Dichtkante des Stößels 41 ist dabei derart ausgestaltet, dass dieses planseitig dichtet. Dies hat den Vorteil, dass der Stößel 41 nicht exakt das Zentrum der Dichtungsbohrung treffen muss. Im geschlossenen Zustand ist ferner das Rückschlagventil der Dichtung aktiviert, sodass ein Zurückfließen des Mediums verhindert wird.

Die erfindungsgemäße Lösung eröffnet dementsprechend in einfacher Weise die Möglichkeit, verschiedene Medien für unterschiedliche Instrumente freizuschalten. Von Vorteil hierbei ist, dass keine Vermischung der Medien stattfindet, da diese bis zum Instrument getrennt geführt sind. Das System kann ohne Weiteres auf zusätzliche Instrumentenplätze oder zusätzliche Medien erweitert werden. Dies ist nur mit sehr geringen Mehrkosten verbunden. Ferner kann eine schnelle Montage erfolgen, da keine Schläuche zu dem jeweiligen Pflegeplatz zu verlegen sind. Stattdessen kann ein zentraler Einspeisungspunkt für das jeweilige Medium genutzt werden.

Anzumerken ist abschließend, dass anstelle der Magnetansteuerung auch eine Anordnung genutzt werden könnte, bei der der Stößel direkt von dem Schieber betätigt wird. In diesem Fall wäre es allerdings erforderlich, den Stößel zusätzlich abzudichten.

## Patentansprüche

1. Wiederaufbereitungsgerät (1) zum Desinfizieren, Sterilisieren und/oder Pflegen von ärztlichen, insbesondere zahnärztlichen Instrumenten (4) mit
einer Verteilereinrichtung (30) zum Zuführen von Reinigungs- oder Pflegemedien zu den Instrumenten (4), wobei die Verteilereinrichtung (30) für jedes Medium einen zentralen Zugang aufweist, der jeweils über ein Ventil (20) mit einer Halterung (5) für ein Instrument (4) verbunden ist,
und wobei jedes Ventil (20) ein Dichtelement (25) aufweist, das durch ein wahlweise positionierbares Betätigungselement (26) der Verteilereinrichtung (30) in eine das Ventil öffnende Stellung bringbar ist,
**dadurch gekennzeichnet,**
**dass** das Betätigungselement (26) an einem Schieber angeordnet ist,
wobei das Dichtelement (25) eines Ventils (20) aus einem ferritischen Material besteht und das Betätigungselement (26) durch einen Magneten gebildet ist, der wahlweise an der Oberseite einer Verteilerplatte positioniert werden kann, wobei die Positionierung des Magneten über den Schieber erfolgt.

2. Wiederaufbereitungsgerät nach Anspruch 1,
**dadurch gekennzeichnet,**
**dass** das Betätigungselement (26) durch einen Elektromagneten gebildet ist.

3. Wideraufbereitungsgerät nach einem der vorherigen Ansprüche,
**dadurch gekennzeichnet,**
**dass** das Dichtelement (25) durch einen Stößel gebildet ist.

4. Wideraufbereitungsgerät nach einem der Ansprüche 1 oder 2,
**dadurch gekennzeichnet,**
**dass** das Dichtelement (25) durch eine Platte gebildet ist.

## Claims

1. A reconditioning device (1) for disinfecting, sterilising and/or maintaining medical, in particular dental, instruments (4), having a distributor unit (30) for supplying cleaning or maintenance media to the instruments (4), wherein the distributor unit (30) has for each medium a central inlet which is connected, in each case, via a valve (20) to a support (5) for an instrument (4),
and wherein each valve (20) has a sealing element (25) which can be brought into a valve-opening position by means of a selectively positionable actuating element (26) of the distributor unit (30),
**characterised in that**
the actuating element (26) is arranged on a slider, wherein the sealing element (25) of a valve (20) is made of a ferritic material, and the actuating element (26) is formed by a magnet, which can be selectively positioned on the upper side of a distributor plate, wherein the positioning of the magnet is effected by way of the slider.

2. A reconditioning device according to claim 1,
**characterised in that**
the actuating element (26) is formed by an electromagnet.

3. A reconditioning device according to one of the preceding claims,
**characterised in that**
the sealing element (25) is formed by a plunger.

4. A reconditioning device according to one of claims 1 or 2,
**characterised in that**
the sealing element (25) is formed by a plate.

## Revendications

1. Appareil de retraitement (1) pour la désinfection, la stérilisation et/ou l'entretien d'instruments (4) médicaux, en particulier dentaires avec un dispositif de distribution (30) pour l'amenée de milieux de nettoyage ou d'entretien aux instruments (4), le dispositif de distribution (30) présentant pour chaque milieu un accès central qui est respectivement relié à une fixation (5) pour un instrument (4) par l'intermédiaire d'une soupape (20),
et chaque soupape (20) présentant un élément d'étanchéité (25) pouvant être amené dans une position ouvrant la soupape par un élément d'actionnement positionnable au choix (26) du dispositif de distribution (30),
**caractérisé en ce que**
l'élément d'actionnement (26) est disposé contre un tiroir,
l'élément d'étanchéité (25) d'une soupape (20) étant composé d'un matériau ferritique et l'élément d'actionnement (26) étant formé par un aimant qui peut être positionné au choix sur la face supérieure d'une plaque de distribution, le positionnement de l'aimant ayant lieu par l'intermédiaire du tiroir.

2. Appareil de retraitement selon la revendication 1,
**caractérisé en ce que**
l'élément d'actionnement (26) est formé par un électroaimant.

3. Appareil de retraitement selon l'une des revendications précédentes,
**caractérisé en ce que**
l'élément d'étanchéité (25) est formé par un poussoir.

4. Appareil de retraitement selon l'une des revendications 1 ou 2,
**caractérisé en ce que**
l'élément d'étanchéité (25) est formé par une plaque.
